# EUROPEAN PATENT SPECIFICATION

(11) **EP 4 252 805 B1**
(45) Date of publication and mention of the grant of the patent: **31.12.2025**
(21) Application number: 21897680.1
(22) Date of filing: 05.11.2021
(51) Int. Cl.: A61M 15/00, A61M 11/00

(54) **INHALATION AID**
INHALATIONSHILFE
AIDE À L'INHALATION

(30) Priority: 27.11.2020 JP 2020197374
(43) Date of publication of application: 04.10.2023
(73) Proprietor: Nippharma Co., Ltd., Tokyo 104-0031 (JP)
(72) Inventor: ONO, Shinichi, Tokyo 104-0031 (JP)
(74) Representative: Peters, Sebastian Martinus
(86) International application number: PCT/JP2021/040885
(87) International publication number: WO 2022/113705

(56) References cited:
- WO-A1-01/00262
- WO-A1-03/089036
- FR-B1- 2 888 510
- GB-A- 2 000 555
- GB-A- 2 344 533
- JP-A- H01 501 290
- JP-A- S57 500 364
- US-A- 4 953 545
- US-A- 6 073 629

## Description

### TECHNICAL FIELD

The present invention relates to an inhalation aid mounted on a spout port of an atomization type inhaler for assisting inhalation of dispersed drugs.

### BACKGROUND ART

As treatment for bronchial asthma and chronic obstructive pulmonary disease (COPD), which are respiratory tract diseases, suction therapy is one of the most effective treatment methods. In the current suction therapy, a Dry Powder Inhaler (DPI) and a Metered-Dose Inhaler (MDI) are generally used for inhaling particulate drugs including, for example, an inhaled corticosteroid, a long-acting β2 agonist, and a long-acting muscarinic antagonist.

Of these, the Metered-Dose Inhaler (MDI) is a type of inhaler for atomizing constant amounts of drug particles using gas pressure. As a patient inhales the drug particles dispersed from the atomization type inhaler, the drug particles get transported to the human respiratory tract through the respiratory organs. However, such atomization type inhaler requires matching the timings of the drug atomization and the patient inhalation. Therefore, in a case where the timing of the drug atomization is difficult to take, such as when the patient is an infant or an old person, an inhalation aid is sometimes attached to a spout port of the atomization type inhaler in order to further ensure patient inhalation of the drug particles. As the inhalation aid (also referred to as an inhalation spacer), ones disclosed in PLT 1 and PLT 2 are known as examples.
Patent Document 1: JP-A-8-266626
Patent Document 2: JP-T-2008-516658
It is noted that GB 2 000 555 A discloses an oral applicator for use together with a pressurized aerosol medicament container, US 6. 73, 629 discloses a spacer for use with a powdered medicament inhaler, and FR 888 510 discloses a system for generating medical aerosols for administration to the airways.

### DISCLOSURE OF THE INVENTION

### PROBLEMS TO BE SOLVED BY THE INVENTION

PLT 1 discloses a device that performs vaporization and dispersion of aerosol droplets for drug inhalation therapy. The device includes a main body having rotational symmetry, a tubular member arranged on one of front ends and protruding by a defined length into the main body, and an intake air member through which ambient air can flow into the main body when a patient inhales the aerosol stored in the main body via the other tubular member. With such configuration, it is considered that, since two spiral turbulences are formed one inside and the other outside in the main body, the outer one of the turbulences avoids an impaction of the drug particles against the inside wall of the vessel. However, in the device in PLT 1, an introduction port of the drug particles dispersed from the atomization type inhaler and an inhalation port for inhaling the drug particles are disposed in a straight line. Although the device in PLT 1 allows shifting the timing of dispersion by the atomization type inhaler from the inhalation timing just as much as the length of the main body, a large amount of the drug particles dispersed into the main body through the introduction port directly heads to the inhalation port. Therefore, the drug particles are required to be inhaled immediately after the drug particles are dispersed into the main body by the atomization type inhaler, and the timing adjustment effect is not in sufficient. Accordingly, a patient with poor inhalation ability may possibly still find it difficult to inhale the drug particles.

Further, PLT 2 discloses a device that is a spacer device for oral administration of a volatile medium containing drug particles. The spacer device includes a chamber having an introduction port, an outlet to be received in the mouth, and a butterfly valve. By thus providing a valve structure in the spacer, a patient can inhale the drug particles dispersed into a main body from an atomization type inhaler at an arbitrary timing, which makes it possible even for a patient with poor inhalation ability to inhale the drug particles relatively easily. However, since the whole structure of the spacer becomes complicated by providing the valve structure in the spacer, in a case where the spacer is a reusable type, there is a problem that disassembly, cleaning, and assembly of the spacer become difficult. In addition, in a case where the spacer is a disposable type, there is a problem that providing the valve structure raises manufacturing cost of the spacer and increases financial burden of the patient who uses the spacer.

Therefore, a main object of the present invention is to provide an inhalation aid that allows a patient to arbitrarily adjust a drug atomization timing with ease without relying on a valve structure.

### SOLUTIONS TO THE PROBLEMS

The present invention is defined by the appended claims, and relates to an inhalation aid 100 for being mounted on a spout port of an atomization type inhaler. The inhalation aid 100 according to the present invention is applicable to a general Metered-Dose Inhaler (MDI). The inhalation aid 100 of the present invention includes a main body part 10, an introduction port 20, a wall surface part 30, and an inhalation port 40. The main body part 10 has a tubular structure, and drug particles are dispersed into an internal space of the main body part 10 from the atomization type inhaler. The main body part 10 has a reverse-tapered shape like as circular-truncated cone shape but may be any other polygonal shape including a triangular tubular shape, a rectangular tubular shape, or a pentagonal tubular shape. The introduction port 20 is an opening portion for introducing the drug particles dispersed from the spout port of the inhaler into the main body part 10, and is disposed on one end side of the main body part 10. The wall surface part 30 is disposed in a position on another end side of the main body part 10 opposed to the introduction port 20. Therefore, the main body part 10 has a tubular structure with a closed bottom in which the introduction port 20 is disposed on the one end side, and the wall surface part 30 is disposed on the other end side. The introduction port 20 and the wall surface part 30 are disposed in a straight line. The inhalation port 40 is an opening portion for inhaling the drug particles introduced into the main body part 10, and is disposed on a side surface 11 of the main body part 10.

As in the above-described configuration, by providing the wall surface part 30 in a position opposed to the introduction port 20 and providing the inhalation port 40 in a position not coaxial with the introduction port 20, dispersed airflows collide with one another, which make it difficult for the drug particles to be introduced from the introduction port 20 to directly reach the inhalation port 40. Therefore, without providing a valve mechanism like the conventional spacer, a patient can arbitrarily adjust the drug inhalation timing with more ease. In addition, a patient who has difficulty inhaling the whole amounts of drugs at once can divide the drugs introduced in the main body part 10 and inhale it over multiple times. To explain more specifically, among the drug particles dispersed into the main body part 10, the drug particles that did not collide with the side surface 11 or the wall surface part 30 of the main body part 10 and are not trapped or accumulated in those areas remain in the main body part 10 for a while after being atomized. These drug particles that remain and are dispersed in the gas phase are transported from the inhalation port 40 installed in a side surface direction perpendicular to the center axis of the introduction port 20 to the human respiratory tract through the respiratory organs by multiple times of oral inhalation. Thus, in the present invention, causes of collision, accumulation, entrapment, and the like into the inner surface of the main body part 10 at the time of atomizing can be eliminated by the drug inhaled structure that is different from the conventional technique. In other words, while the conventional technique allows accumulation/entrapment rate due to collision to be changed by influence of inhalation conditions (such as a respiratory rate or a tidal volume) at the time of inhalation, in the present invention, the drug atomization rate does not receive strong influence of the inhalation conditions even in normal breathing.

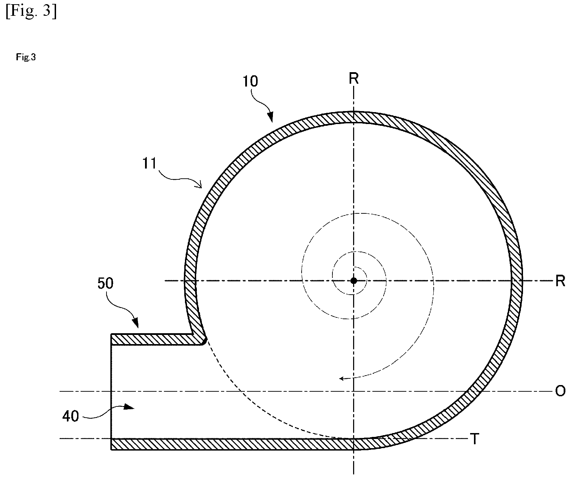

Especially, in the inhalation aid 100 according to the present invention, the inhalation port 40 is extending along a tangential direction of the main body part 10. That is, in a case where the main body part 10 is formed as a cylindrical shape, when viewed from the wall surface part 30 side (or the introduction port 20 side) of the inhalation aid 100, the inhalation port 40 does not extend along a radial direction of the main body part 10, and instead, extends along the tangential direction of the cylindrical main body part 10. By thus forming the inhalation port 40 in a shifted position with respect to the radial direction of the main body part 10, when the drug particles in the main body part 10 are inhaled via the inhalation port 40, a spiral airflow can be generated in the main body part 10 (see Fig. 3). Accordingly, an inhalation efficiency of the drug particles dispersed into the main body part 10 can be improved.

In the inhalation aid 100 according to the present invention, the inhalation port 40 is preferred to be disposed approximately on an extended line of the wall surface part 30. In the present invention, while the inhalation port 40 can be disposed on the side surface 11 of the main body part 10 between the introduction port 20 and the wall surface part 30, it is especially preferred to be disposed in a position near the wall surface part 30, particularly on the extended line of the wall surface part 30. With such configuration, the drug particles dispersed from the introduction port 20 toward the wall surface part 30 can be efficiently inhaled.

In the inhalation aid 100 according to a preferred embodiment, the inner surface inside the main body part 10 of the wall surface part 30 may be formed as a recessed surface. By thus forming the inner surface of the wall surface part 30 as a recessed surface (especially a concave surface), the flow of the drug particles inversing on the wall surface part 30 can be focalized in the center axis direction. Accordingly, the drug particles become less likely to be trapped or accumulated in the inner surface of the main body part 10 and, as a result, the drug inhalation rate improves. That is, while drug particles are dispersed radially, in a straight line, and intermittently from the atomization type inhaler into the main body part 10 of the inhalation aid 100, which causes the drug particles to collide with the side surface 11 and the wall surface part 30 inside the main body part 10, and at least part of the drug particles to be accumulated or trapped in those areas, the present invention proposes a method for reducing such accumulation and entrapment of the drug particles.

In the inhalation aid 100 according to a preferred embodiment, one or a plurality of protrusions or recesses may be formed on the inner surface inside the main body part 10 of the wall surface part 30. The protrusions or recesses include dot shaped ones and linear ones. (Linear-shaped protrusions or recesses are also referred to as having a ridge shape or a groove shape.) The drug particles dispersed into the main body part 10 of the inhalation aid 100 repel and/or collide, or become accumulated or trapped at boundary layers of the side surface 11 and the wall surface part 30 after being atomized, and especially, a complicated turbulent flow is formed inside the main body part 10 by collision and merging of the flow of the drug particles that have repelled in the wall surface part 30 and the flow of the subsequently dispersed drug particles. In this respect, the flow of the drug particles can be rectified by forming, for example, a mesh structure with a plurality of protrusions or recesses on the inner surface of the wall surface part 30. Further, in order to suppress the turbulent flow of the drug particles, it is preferred to form a plurality of protrusions or recesses on the inner surface of the wall surface part 30 to rectify the flow and also, as described above, form the inner surface of the wall surface part 30 as a recessed surface to focalize the inversed flow of the drug particles in the center axis direction.

In the inhalation aid 100 according to the present invention, the side surface 11 (especially the inner surface of the side surface 11) of the main body part 10 is at least partly formed in a reverse-tapered shape having a diameter that expands from one end side toward another end side. The drug particles dispersed into the main body part 10 are dispersed in the main body part 10 while radially expanding, but by forming the side surface 11 of the main body part 10 as a reverse-tapered shape, an atomization flow of the drug particles in the direction of the main body part 10 becomes reduced and allows suppressing the drug particles being attached to or trapped on the side surface 11.

### ADVANTAGEOUS EFFECTS OF THE INVENTION

The present invention can provide an inhalation aid with which a patient may arbitrarily adjust a drug inhalation timing with ease without relying on a valve structure.

### BRIEF DESCRIPTION OF THE DRAWINGS

Fig. 1 is a perspective view illustrating one embodiment of an inhalation aid.
Fig. 2 is a cross-sectional view schematically illustrating a vertical cross-sectional surface of the inhalation aid.
Fig. 3 is a cross-sectional view schematically illustrating a lateral cross-sectional surface of the inhalation aid.
Fig. 4 illustrates an example of a rectification structure on an inner surface of a wall surface part.
Fig. 5 illustrates an example of a focalization structure on the inner surface of the wall surface part

### DESCRIPTION OF PREFERRED EMBODIMENTS

The following describes embodiments to embody the present invention using the drawings. The present invention is not limited to the embodiments described below, but includes those appropriately modified from the embodiments below by a person skilled in the art within the scope of the claims.

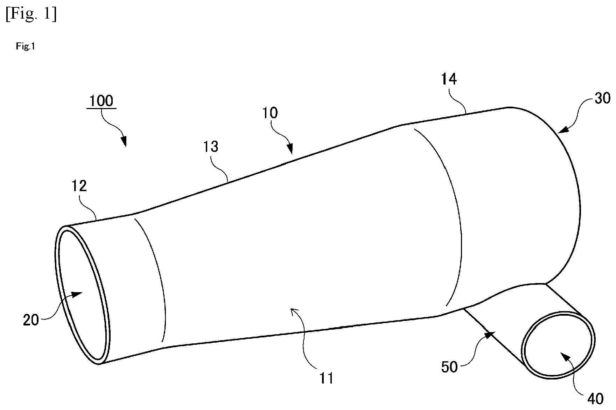

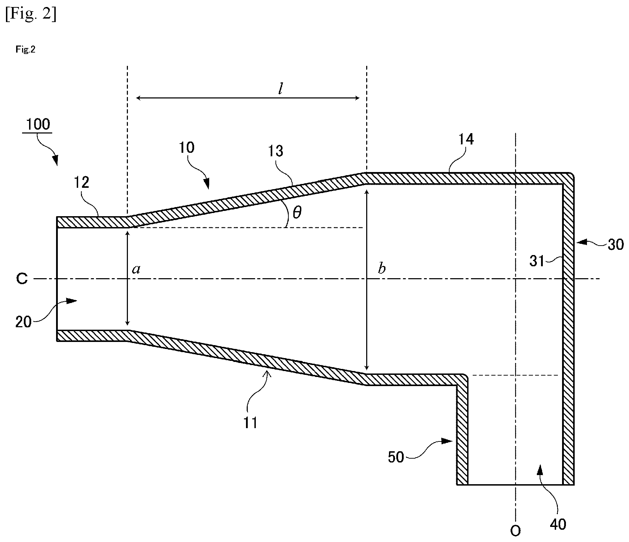

Fig. 1 to Fig. 3 illustrate one embodiment of an inhalation aid 100 according to the present invention. Fig. 1 is a perspective view illustrating an overall configuration of the inhalation aid 100, Fig. 2 is a cross-sectional view in a longitudinal direction, and Fig. 3 is a cross-sectional view in a lateral direction. As shown in these drawings, the inhalation aid 100 is configured as a tubular shape with a closed bottom in which an introduction port 20 is disposed at one end of the cylindrical main body part 10 and a wall surface part 30 is disposed at another end side of the main body part 10. A side surface 11 is formed from one end to the other end of the main body part 10, and a mouthpiece 50 having an inhalation port 40 is disposed on the side surface 11 near the wall surface part 30. The internal space of the main body part 10 communicates with the introduction port 20 and the inhalation port 40 of the mouthpiece 50. Therefore, a patient can inhale drug particles introduced from the introduction port 20 to inside of the main body part 10 by breathing while holding the mouthpiece 50 in the mouth.

As the introduction port 20 of the inhalation aid 100, a spout port of a publicly known Metered-Dose Inhaler (MDI) can be attached. Examples of the currently publicly known MDIs include Adoair (registered trademark) Aerosol and Flutiform (registered trademark), but MDIs applicable to the present invention are not limited to these.

The inhalation aid 100 may be either a reusable type or a disposable type. A material that constitutes the inhalation aid 100 is not particularly limited, and as a reusable type, it may be made of plastic, carbon, or metal, and as a disposable type, it may be made of paper or wood. Since the inhalation aid 100 of the present invention is structurally simple, it can be easily washed and has excellent maintainability. Moreover, since its manufacturing cost can be kept at a low price, both a reusable type and a disposable type can be preferably used.

As shown in Fig. 1 and Fig. 2, the main body part 10 can be separated into a side parallel part of introduction port 12, a reverse-tapered part 13, and a side parallel part of wall surface port 14. That is, inner surfaces of the side surface 11 of the main body part 10 in the side parallel part of introduction port 12 and the side parallel part of wall surface port 14 are parallelly formed, viewed from a cross-sectional surface, and the reverse-tapered part 13 is positioned between these parallel parts 12, 14. However, when comparing an inner diameter a (diameter) of the side parallel part of introduction port 12 and an inner diameter b (diameter) of the side parallel part of wall surface port 14, the inner diameter b is formed larger (a < b). Therefore, the reverse-tapered part 13 positioned between the parallel parts 12, 14 is configured to have a predetermined length *l* such that an inner diameter of the side surface 11 radially expands gradually from the side parallel part of introduction port 12 toward the side parallel part of wall surface port 14.

In Fig. 2, an inclination of the inner surface of the side surface 11 constituting the reverse-tapered part 13 is indicated by reference numeral ***θ***. The inclination ***θ*** referred to here is a half value of a taper angle. The inclination ***θ*** [%] of the side surface 11 constituting the reverse-tapered part 13 can be obtained by a formula: **(*b*** - ***a***)/l × 0.5. Specifically, the inner diameter ***a*** of the side parallel part of introduction port 12 is preferred to be 30 to 35 mm such that the spout port of the MDI can be appropriately attached. Further, the inner diameter ***b*** of the side parallel part of wall surface port 14 is preferred to be 45 to 50 mm so as to ensure enough space for the drug particles to spread efficiently. Further, the length ***l*** of the reverse-tapered part 13 is preferred to be 80 to 180 mm so as to ensure enough time and length for the drug particles introduced from the introduction port 20 to spread. As a result, the inclination ***θ*** [degree] of the inner surface of the side surface 11 constituting the reverse-tapered part 13 is preferred to be in a range of 5 to 15 degrees.

As shown in Fig. 2, in the inhalation aid 100, the wall surface part 30 is disposed in a position opposed to the introduction port 20. Specifically, in Fig. 2, a center line C that passes through the center of the introduction port 20 and extends along the longitudinal direction of the main body part 10 is illustrated, and the wall surface part 30 is arranged in a position intersecting with the center line C. In particular, the wall surface part 30 is preferred to be perpendicular to the center line C of the introduction port 20. Accordingly, it is considered that at least a part of the drug particles dispersed from the introduction port 20 toward the main body part 10 collide with the wall surface part 30 and are reflected in the internal space side of the main body part 10. Further, in a case where the main body part 10 has a cylindrical shape, the wall surface part 30 becomes to have a circular shape. In particular, the main body part 10 and the wall surface part 30 are configured in a concentric circle.

As shown in Fig. 1 to Fig. 3, the mouthpiece 50 having the inhalation port 40 is formed in the side surface 11 of the main body part 10. The mouthpiece 50 is preferred to have a shape that is easy to be held in the mouth, such as an ellipse, a perfect circle, or the like. The mouthpiece 50 is preferred to be disposed in the side parallel part of wall surface port 14 of the main body part 10, and especially preferred to be disposed along an extended line of the wall surface part 30. Further, as shown in Fig. 2, a level difference and the like are preferred not to exist in the internal space between the wall surface part 30 and the mouthpiece 50. This allows the flow of the drug particles near the wall surface part 30 to smoothly reach the outlet of the inhalation port 40 at the time of inhalation. If a level difference exists in the internal space inside the main body part 10, the drug particles may possibly remain or get stuck in that part, and thus, such level difference should be avoided as much as possible. However, there is no problem even if a level difference and the like exists in the internal space between the wall surface part 30 and the mouthpiece 50 as long as the flow of the drug particles are directed toward the inhalation port 40. For example, a level difference of around 5 to 15 mm is acceptable.

Further, the inhalation port 40 of the mouthpiece 50 is preferred to be perpendicular to the internal space of the main body part 10. Specifically, Fig. 2 illustrates the center line O that passes through the center of the inhalation port 40 and extends along the mouthpiece 50, and this center line O of the inhalation port 40 is preferred to be perpendicular to the above-described center line C of the introduction port 20. Further, the center line O of the inhalation port 40 is preferred to extend in parallel with the wall surface part 30. Note that, the center line O of the inhalation port 40 and the center line C of the introduction port 20 are not necessarily required to be perpendicular to one another in the present invention, and may be an angle designed arbitrarily. For example, the corner made by the center lines O, C is preferred to have an angle of 60 to 120 degrees, and is particularly preferred to have an angle of 80 to 100 degrees. Further, in a case where the center lines O, C are not perpendicular to one another, the center line C and the wall surface part 30 also need not be perpendicular to one another. In this case, maintaining a parallel relationship between the center line O and the wall surface part 30 is prioritized, and the corner made by the center line C and the wall surface part 30 can have an angle in a range of 80 to 100 degrees.

Further, as shown in Fig. 3, the inhalation port 40 of the mouthpiece 50 is preferred to extend along the tangential direction of the main body part 10. That is, in Fig. 3, a radial direction of an inner circumference of the cylindrical main body part 10 is indicated by the radial direction R, the center line O of the inhalation port 40 of the mouthpiece 50 does not coincide with the radial direction R of the main body part 10. Note that, while the center line O of the inhalation port 40 becomes parallel with respect to one of straight lines extending in the radial direction R of the main body part 10, the center line O of the inhalation port 40 is shifted to one side of the straight line extending in the radial direction R and does not overlap with the line. On the other hand, in Fig. 3, the tangential direction of the inner circumference of the cylindrical main body part 10 is indicated by reference numeral T. The tangent line T of the inner circumference is a straight line in parallel with respect to the radial direction R extending along a certain radial direction of the inner circumference. Note that, the tangent line T of the inner circumference and the radial direction R extending along the radial direction do not overlap, and these two straight lines are always a constant distance apart. In this case, the center line O of the inhalation port 40 of the mouthpiece 50 extends in parallel with the tangential direction T of the main body part 10. By thus shifting the inhalation port 40, the spiral flow as indicated in Fig. 3 can be generated in the internal space of the main body part 10 at the time of inhalation. As a result, the inhalation efficiency of the drugs can be improved.

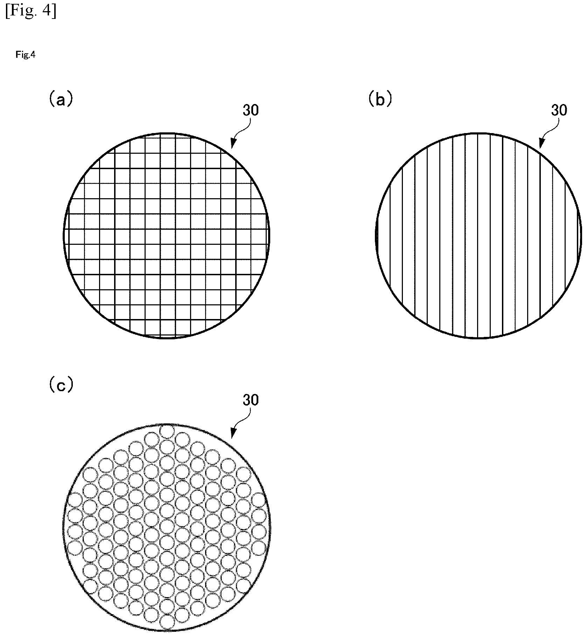

Fig. 4 illustrates an example of a rectification structure of an inner surface 31 of the wall surface part 30. In this rectification structure, a plurality of linear or dot shaped protrusions or recesses are disposed in the inner surface 31 of the wall surface part 30. In the example indicated in Fig. 4(a), a plurality of linear protrusions (ridges) or recesses (grooves) are formed in a grid form (mesh form). That is, in the grid pattern indicated in Fig. 4(a), a plurality of linear protrusions or recesses extending in one direction and a plurality of linear protrusions or recesses extending in a direction intersecting therewith are formed. On the other hand, in the example shown in Fig. 4(b), a plurality of linear protrusions or recesses are formed in a stripe form. That is, in the stripe pattern indicated in Fig. 4(b), a plurality of linear protrusions or recesses extend in parallel only in one direction. Further, in the example indicated in Fig. 4(c), a plurality of dot-shaped protrusions or recesses are arranged at predetermined intervals. Respective sizes of the protrusions or recesses and the intervals (pitches) of the protrusions or recesses can be appropriately adjusted according to characteristics of the drug particle dispersed airflow, the particle diameter of the drugs introduced into the main body part 10, and the like. As another feature of the inhalation aid 100 of the present invention, the wall surface part 30 is disposed in a position opposed to the introduction port 20, and the rectification structure as indicated in Fig. 4 is applicable to the inner surface 31 of this wall surface part 30.

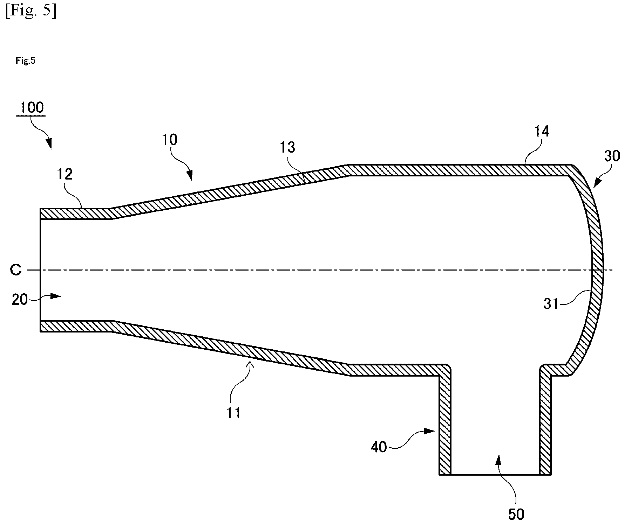

Fig. 5 illustrates an example of a focalization structure of the inner surface 31 of the wall surface part 30. As shown in Fig. 5, the inner surface 31 of the wall surface part 30 can be a surface recessed as a recessed surface. In a case where the main body part 10 has a cylindrical shape, the wall surface part 30 is configured as a circular shape, and the inner surface 31 of the wall surface part 30 similarly becomes a surface recessed in a hemispherical shape. Especially, as indicated in Fig. 5, the deepest portion of the recessed surface of the wall surface part 30 is preferred to be positioned on the extended line of the center line C of the introduction port 20. By thus forming the inner surface 31 of the wall surface part 30 as a recessed surface, the drug particles that have collided with the wall surface part 30 are inversed toward the center of the main body part 10. As mentioned above, when the drug particles are introduced into the main body part 10, the drug particles advance toward the wall surface part 30 while radially spreading. However, even if the wall surface part 30 remains a planar surface, the drug particles that are reflected by the wall surface part 30 are redispersed in a direction colliding with the side surface 11 of the main body part 10. Further, by focalizing the flow of the drug particles by forming the wall surface part 30 as a recessed surface, it is possible to suppress the drug particles reflected by the wall surface part 30 being trapped on the inner surface of the side surface 11.

In addition, the rectification structure illustrated in Fig. 4 and the focalization structure illustrated in Fig. 5 can be used together. That is, the inner surface 31 of the wall surface part 30 may be molded as a recessed surface (especially a concave surface), and a plurality of protrusions or recesses can be formed on that recessed surface.

Note that, the inner surface 31 of the wall surface part 30 can also be bulged as a convex surface in opposed to the example indicated in Fig. 5. Although this case is not preferrable since it allows the drug particles reflected by the inner surface 31 of the wall surface part 30 to easily collide with the side surface 11 of the main body part 10. If a certain meaning is found in forming the inner surface 31 of the wall surface part 30 as a convex surface, it may be configured as such.

In this application, the embodiments of the present invention have been described above by referring to the drawings to express the contents of the present invention. However, the present invention is not limited to the embodiments described above, and includes changed embodiments and improved embodiments that fall within the scope of the claims.

### DESCRIPTION OF REFERENCE SIGNS

10... main body part
11... side surface
12... side parallel part of introduction port
13... reverse-tapered part
14... side parallel part of wall surface port
20... introduction port
30... wall surface part
31... inner surface
40... inhalation port
50... mouthpiece
100... spacer

## Claims

1. An inhalation aid (100) for being mounted on a spout port of an atomization type inhaler, comprising:
a tubular main body part (10);
an introduction port (20) disposed on one end side of the main body part (10) for introducing drugs atomized from the spout port of the inhaler into the main body part (10);
a wall surface part (30) disposed in a position opposed to the introduction port (20) on another end side of the main body part (10);
a side surface (11) formed from the one end side to the other end side of the main body part (10), wherein the side surface (11) is formed at least partly in a reverse-tapered shape radially expanding from the one end side to the other end side; and
an inhalation port (40) disposed on the side surface (11) for inhaling the drugs introduced into the main body part (10), wherein
the inhalation port (40) extends along a tangential direction (T) of the main body part (10), and
while a center line (O) of the inhalation port (40) is parallel to one of the straight lines extending in a radial direction (R) of the main body part (10), the center line (O) of the inhalation port (40) is shifted to one side of the straight line extending in the radial direction (R) and does not overlap with it, so that a spiral airflow can be generated in the main body part (10) when the drug particles in the main body part (10) are inhaled via the inhalation port (40).

2. The inhalation aid according to claim 1, wherein the inhalation port (40) is disposed on an extended line of the wall surface part (30).

3. The inhalation aid according to claim 1, wherein an inner surface inside the main body part (10) of the wall surface part (30) is formed as a recessed surface.

4. The inhalation aid according to claim 1, wherein one or a plurality of protrusions or recesses are formed on an inner surface inside the main body part (10) of the wall surface part (30).

## Patentansprüche

1. Inhalationshilfe (100) zur Befestigung an einer Mündungsöffnung eines Zerstäuber-Inhalators, umfassend:
einen rohrförmigen Hauptkörperabschnitt (10);
eine Einführungsöffnung (20), die an einer Endseite des Hauptkörperabschnitts (10) angeordnet ist, zum Einführen von zerstäubten Wirkstoffen von der Mündungsöffnung des Inhalators in den Hauptkörperabschnitt (10);
ein Wandflächenabschnitt (30), der in einer Position gegenüberliegend der Einführungsöffnung (20) an einer anderen Endseite des Hauptkörperabschnitts (10) angeordnet ist;
eine Seitenfläche (11), die von der einen Endseite bis zu der anderen Endseite des Hauptkörperabschnitts (10) ausgebildet ist, wobei die Seitenfläche (11) zumindest teilweise in einer gegenkonischen Form ausgebildet ist, die sich von der einen Endseite zu der anderen Endseite radial erweitert; und
eine Inhalationsöffnung (40), die an der Seitenfläche (11) angeordnet ist, zur Inhalation der in den Hauptkörperabschnitt (10) eingeführten Wirkstoffe,
wobei die Inhalationsöffnung (40) sich entlang einer Tangentialrichtung (T) des Hauptkörperabschnitts (10) erstreckt, und
während eine Mittellinie (O) der Inhalationsöffnung (40) parallel zu einer der sich in Radialrichtung (R) des Hauptkörperabschnitts (10) erstreckenden Geraden ist, ist die Mittellinie (O) der Inhalationsöffnung (40) zu einer Seite der sich in Radialrichtung (R) erstreckenden Geraden versetzt und überlappt diese nicht, so dass ein spiralförmiger Luftstrom im Hauptkörperabschnitt (10) erzeugt werden kann, wenn die Wirkstoffpartikel in dem Hauptkörperabschnitt (10) über die Inhalationsöffnung (40) inhaliert werden.

2. Inhalationshilfe gemäß Anspruch 1, wobei die Inhalationsöffnung (40) auf einer verlängerten Linie des Wandflächenabschnitts (30) angeordnet ist.

3. Inhalationshilfe gemäß Anspruch 1, wobei eine Innenfläche innerhalb des Hauptkörperabschnitts (10) des Wandflächenabschnitts (30) als vertiefte Fläche ausgebildet ist.

4. Inhalationshilfe gemäß Anspruch 1, wobei eine oder eine Vielzahl von Erhebungen oder Vertiefungen auf einer Innenfläche innerhalb des Hauptkörperabschnitts (10) des Wandflächenabschnitts (30) ausgebildet sind.

## Revendications

1. Dispositif d'aide à l'inhalation (100) destiné à être monté sur un orifice de pulvérisation d'un inhalateur de type à atomisation, comprenant:
une partie de corps principal tubulaire (10);
un orifice d'introduction (20) disposé sur un côté d'extrémité de la partie de corps principal (10) pour introduire des médicaments atomisés à partir de l'orifice de pulvérisation de l'inhalateur dans la partie de corps principal (10);
une partie de surface de paroi (30) disposée dans une position opposée à l'orifice d'introduction (20) sur un autre côté d'extrémité de la partie de corps principal (10); et
une surface latérale (11) formée du côté d'extrémité précité à l'autre côté d'extrémité de la partie de corps principal (10), la surface latérale (11) étant formée au moins en partie suivant une forme de cône inversé s'étendant radialement du côté d'extrémité précité à l'autre côté d'extrémité; et
un orifice d'inhalation (40) disposé sur la surface latérale (11) pour inhaler les médicaments introduits dans la partie de corps principal (10), dans lequel
l'orifice d'inhalation (40) s'étend le long d'une direction tangentielle (T) de la partie de corps principal (10), et
tandis qu'une ligne centrale (O) de l'orifice d'inhalation (40) est parallèle à l'une des lignes droites s'étendant dans une direction radiale (R) de la partie de corps principal (10), la ligne centrale (O) de l'orifice d'inhalation (40) est décalée vers un côté de la ligne droite s'étendant dans la direction radiale (R) et ne la chevauche pas, de telle sorte qu'un flux d'air en spirale peut être généré dans la partie de corps principal (10) lorsque les particules de médicament dans la partie de corps principal (10) sont inhalées par l'orifice d'inhalation (40).

2. Dispositif d'aide à l'inhalation selon la revendication 1, dans lequel l'orifice d'inhalation (40) est disposé sur une ligne prolongée de la partie de surface de paroi (30).

3. Dispositif d'aide à l'inhalation selon la revendication 1, dans lequel une surface interne à l'intérieur de la partie de corps principal (10) de la partie de surface de paroi (30) est formée en tant que surface en retrait.

4. Dispositif d'aide à l'inhalation selon la revendication 1, dans lequel une ou plusieurs protubérances ou un ou plusieurs renfoncements sont formé(e)s sur une surface interne à l'intérieur de la partie de corps principal (10) de la partie de surface de paroi (30).
